# EUROPÄISCHE PATENTSCHRIFT

(11) **EP 0 866 117 B1**
(45) Veröffentlichungstag und Bekanntmachung des Hinweises auf die Patenterteilung: **06.05.2004**
(21) Anmeldenummer: 98104558.6
(22) Anmeldetag: 13.03.1998
(51) Int. Cl.: C11D 17/00, A61K 9/107, A61K 7/00, A23L 1/30, A23L 1/302

(54) **Mikroemulsion**
Microemulsion
Micro-émulsion

(30) Priorität: 17.03.1997 CH 62897
(43) Veröffentlichungstag der Anmeldung: 23.09.1998
(73) Patentinhaber: DSM IP Assets B.V., 6411 TE Heerlen (NL)
(72) Erfinder: Bauer, Kurt, 79112 Freiburg-Tiengen (DE); Neuber, Clarissa, 78054 Villingen-Schwenningen (DE); Schmid, Axel, 78244 Gottmadingen-Bietingen (DE); Völker, Karl Manfred, 79100 Freiburg (DE)
(74) Vertreter: Müller, Ingrid, Dr.

(56) Entgegenhaltungen:
- GB-A- 2 297 759
- US-A- 5 045 337
- DATABASE WPI Section Ch, Week 8627 Derwent Publications Ltd., London, GB; Class B07, AN 86-172070 XP002069484 & JP 61 103 536 A (POLA KASEI KOGYO KK)
- PATENT ABSTRACTS OF JAPAN vol. 008, no. 145 (C-232), 6.Juli 1984 & JP 59 051214 A (TAIHOU YAKUHIN KOGYO KK;OTHERS: 01), 24.März 1984,
- PATENT ABSTRACTS OF JAPAN vol. 095, no. 011, 26.Dezember 1995 & JP 07 204487 A (YAKUKEN:KK), 8.August 1995,
- DATABASE WPI Section Ch, Week 8706 Derwent Publications Ltd., London, GB; Class B05, AN 87-040923 XP002069485 & JP 62 000 419 A (SHISEIDO CO LTD)
- PATENT ABSTRACTS OF JAPAN vol. 097, no. 010, 31.Oktober 1997 & JP 09 157159 A (LION CORP), 17.Juni 1997,
- PATENT ABSTRACTS OF JAPAN vol. 009, no. 293 (C-315), 20.November 1985 & JP 60 137260 A (KUNOORU SHIYOKUHIN KK), 20.Juli 1985,

## Beschreibung

Die Erfindung betrifft eine Mikroemulsion vom Typ Oel-in-Wasser, enthaltend mindestens einen Polyglycerolester als Emulgator und mindestens einen lipophilen Stoff als innere Phase.

Gewisse Stoffe, die zur Herstellung einer Mikroemulsion verwendet werden können, führen zu Mikroemulsionen, die bei Anwendung in Lebensmitteln oder im Pharma- oder Kosmetikbereich für den Menschen bedenklich sein können. Es wurden beispielsweise im Pharmabereich Mikroemulsionen unter Verwendung von Tensiden als Emulgatoren hergestellt, welche Polyoxyethylenreste im hydrophilen Bereich der Kette enthalten. Mit Hilfe dieser Tenside konnten zwar fettlösliche Stoffe, wie z.B. Vitamine oder pharmazeutische Wirkstoffe, in Wasser solubilisiert werden, sodass eine klare Emulsion entsteht. Allerdings können diese Emulgatoren gesundheitsschädlich sein. Eine Anwendung in Lebensmitteln verbietet sich daher, hauptsächlich weil diese Tenside Restmonomere enthalten, die als potentiell carcinogen gelten.

Aus der Patentpublikation GB 2,222,770 sind Mikroemulsionen mit Zuckerestern als Emulgatoren bekannt, die als notwendige Coemulgatoren jedoch toxische niedermolekulare Polyoxyethylene enthalten.

Ferner sind aus der US-Patentschrift 5,045,337 für den Lebensmittelbereich Wasser-in-Oel-Mikroemulsionssysteme mit Polyglycerolestern oder Mono-Diglyceriden als Emulgatoren zur Formulierung hydrophiler Vitamine und Aromastoffe bekannt.

Die US-Patentschrift 4,835,002 beschreibt Oel-in-WasserMikroemulsionen mit Polyglycerolestern zur Formulierung lipohiler verzehrbarer Oele, Geruchsöle oder Aromaöle, die für den Lebensmittelbereich vorgesehen sind.

Die vorliegende Erfindung betrifft eine beliebig mit Wasser verdünnbare Mikroemulsion vom Typ Oel-in-Wasser mit mindestens einem Triglycerinmonofettsäureester ausgewählt aus der gruppe Triglycerinmonolaurat, Triglycerinmonocaprinat und Triglycerinmonocaprylat als Emulgator und mindestens einem lipophilen Stoff ausgewählt aus der Gruppe Carotinoide, insbesondere β-Carotin, Vitamine A, D, E und K und deren Derivate und mehrfach ungesättigte Fettsäuren wie z.B. Arachidonsäure, Eicosapentaensäure, Docosahexaensäure, als innere Phase.

Derivate der Vitamine sind beispielsweise Vitamin A-Acetat, Vitamin A-Palmitat, Vitamin E-Acetat und dergleichen. Unter Vitamin E wird synthetisches Tocopherol oder eine Mischung natürlicher Tocopherole verstanden.

Mit dieser Mikroemulsion ist es möglich, die genannten lipophilen Stoffe in jeder beliebigen Menge Wasser zu solubilisieren, ohne dass das mikroskopische Erscheinungsbild der Mikroemulsion verändert wird beziehungsweise ohne dass die Mikroemulsion bricht.

Die verwendeten Emulgatoren sind untoxisch, so dass derartige Mikroemulsionen in Lebensmitteln eingesetzt werden können. Sie sind auch im Pharma-Bereich verwendbar. Kosmetische Formulierungen sind ebenfalls möglich.

Bevorzugte Triglycerinmonofettsäureester sind, Triglycerinmonolaurat und Triglycerinmonocaprylat.

Die Herstellung der Mikroemulsionen gelingt wahlweise mit oder ohne Coemulgator. Bevorzugte Coemulgatoren sind: Ethanol, Propylenglycol, Transcutol, Polyethlenglycole, Polyglycerole, Monoglyceride oder Lecithin, insbesondere Transcutol, Monoglyceride oder Lecithin, wobei Lecithin besonders bevorzugt ist.

Die Mikroemulsion kann noch darüber hinaus mindestens ein Trägeröl enthalten. Im Trägeröl wird der lipophile Stoff zumindest teilweise gelöst, was den Gesamtanteil an lipophilem Stoff in der fertigen Mikroemulsion erhöht. Das Trägeröl ist vorzugsweise Orangenöl, Palmöl, ein Triglycerid, Squalan, Squalen, Limonen, Isopropylmyristat, oder Isopropylpalmitat, insbesondere Orangenöl, Squalan, Limonen, bevorzugt Orangenöl.

Die Mikroemulsion enthält als Vitamin insbesondere Tocopherol und/oder Vitamin A, insbesondere Vitamin A-Palmitat.

Als Carotinoid enthält die Mikroemulsion bevorzugt all-trans-β-Carotin, 9-cis-β-Carotin, 13-cis-β-Carotin, Apocarotinal, Astaxantin, Canthaxanthin, Crocetin oder Lycopin. Besonders bevorzugt ist ein β-Carotin.

Bevorzugt liegt der Anteil an lipophilem Stoff, beispielsweise Tocopherol, bei 1-20 Gew.%, an Emulgator bei 10-99 Gew.% und gegebenenfalls an Coemulgator bei 1-89 Gew.%, wobei vorzugsweise das Verhältnis von Emulgator zu Coemulgator 50:50 Gew.% beträgt.

Die Mikroemulsion lässt sich insbesondere in Lebensmitteln, pharmazeutischen Zubereitungen und in Kosmetika verwenden.

Die erfindungsgemässe Mikroemulsion ist besonders geeignet für Formulierungen, in denen die obgenannten lipophilen Stoffe als lipophile pharmazeutische Wirksubstanzen solubilisiert werden sollen. So wurde insbesondere Tocopherol als Lipid verwendet.

Die Mikroemulsion ist mit Wasser in jedem Verhältnis verdünnbar beziehungsweise mischbar. Eine pharmazeutisch zweckmässige Verdünnung der Mikroemulsion enthält z.B. 90 Gew.% Wasser. In dieser Formulierung wären dann noch 0.1-2 Gew.% Tocopherol enthalten.

Die Geschwindigkeit der Mikroemulsionsbildung ist abhängig von der Auflösungsgeschwindigkeit der einzelnen Komponenten. Wenn z.B. salbenartige Emulgatoren, wie beispielsweise Triglycerinmonolaurat, verwendet werden, kann die Lösegeschwindigkeit dieser Emulgatoren beschleunigt werden, indem schwach gerührt und eventuell auf ca. 40-45° C erwärmt wird. Dazu ist ein Magnetrührer geeignet. Es lässt sich aber auch jeder andere Rührer und jedes beliebige Heizsystem verwenden. Die Formulierung ist fertig, wenn aus den einzelnen Komponenten eine klare, isotrope Flüssigkeit entstanden ist. Dies ist nach wenigen Minuten der Fall.

Die Formulierungen können zusätzlich Aromazusätze, Farbstoffe und/oder Verdickungsmittel enthalten, wenn ihre Anwendung dies erfordern sollte. Beträgt der Gehalt an Polyglycerolestern weniger als ca. 3-5%, kann die Formulierung zusätzlich mit gängigen Konservierungsstoffen konserviert werden.

Weitere Merkmale und Einzelheiten ergeben sich aus den nachfolgenden Beispielen.

### Beispiel 1

5 g Tocopherol wurde mit 30 g Triglycerinmonolaurat sowie 65 g demineralisiertem Wasser zusammengewogen und bei 45 - 50°C unter Rühren gelöst. Es entstand eine klare, isotrope Mikroemulsion, die zwischen 4°C und 45°C stabil war. Diese konnte mit jeder beliebigen Menge Wasser verdünnt werden.

### Beispiel 2

1 g Tocopherol wurde mit 4,5 g Triglycerinmonolaurat und 4,5 g Ethanol verrührt. Bei Raumtemperatur entstand eine klare, gelbe Mikroemulsion. Diese konnte mit jeder beliebigen Menge Wasser verdünnt werden, ohne dass sie brach.

### Beispiel 3

1 g Tocopherol wurde mit 4,5 g Triglycerinmonolaurat und 4,5 g Propylenglycol bei Raumtemperatur verrührt. Es entstand eine gelbe, dickflüssige Lösung. Die erhaltene Mikroemulsion konnte mit über 100 ml Wasser verdünnt werden, ohne dass die Mikroemulsion brach.

### Beispiel 4

2.5 g Tocopherol und 7.5 g Triglycerinmonocaprylat wurden mit einem Magnetrührer bei Raumtemperatur gerührt, bis eine klare Mikroemulsionsgrundlage entstand. Diese Grundlage konnte mit Wasser verdünnt werden und bildete dabei Mikroemulsionen vom o/w-Typ.

### Beispiel 5

0,6 Phytomenadion (Vitamin K1), 2.35 g Lecithin (Coemulgator) und 7.05 g Triglycerinmonocaprylat wurden mit einem Magnetrührer bei Raumtemperatur gerührt, bis eine klare Mikroemulsionsgrundlage entstand. Diese Grundlage konnte mit Wasser verdünnt werden und bildete dabei Mikroemulsionen von o/w-Typ.

### Beispiel 6

1 g Tocopherol wurde mit 9 g Triglycerinmonocaprinat bei Raumtemperatur verrührt. Die erhaltene klare, gelbe Mikroemulsion war in jedem Verhältnis mit Wasser mischbar, ohne dass die Mikroemulsion brach.

### Beispiel 7

1.6 g Orangenöl (Trägeröl) wurden mit 3.0 g Triglycerinmonolaurat (Emulgator) und 5.4 g demineralisiertem Wasser zusammengewogen und unter Rühren innerhalb einiger Minuten gelöst. Es entstand eine transparente gelbe Mikroemulsionsgrundlage. Zu dieser Mikroemulsionsgrundlage wurden 37 mg β-Apo-8'-carotinsäureethylester gegeben. Das Carotinoid wurde unter Rühren in der Mikroemulsionsgrundlage innerhalb einiger Stunden gelöst. Es entstand eine klare, rote Mikroemulsion, die mit jeder beliebigen Menge Wasser mischbar war.

### Beispiel 8

1.6 g Orangenöl (Trägeröl) wurden mit 3.0 g Triglycerinmonolaurat (Emulgator) und 5.4 g demineralisiertem Wasser zusammengewogen und unter Rühren innerhalb einiger Minuten gelöst. Zu dieser Mikroemulsionsgrundlage wurden 31 mg β-Apo-8'-carotinal gegeben. Das Carotinoid wurde unter Rühren in der Mikroemulsionsgrundlage innerhalb einiger Stunden gelöst. Es entstand eine klare, rote Mikroemulsion, die mit jeder beliebigen Menge Wasser mischbar war.

### Beispiel 9

1.6 g Orangenöl (Trägeröl) wurden mit 3.0 g Triglycerinmonolaurat (Emulgator) und 5.4 g demineralisiertem Wasser zusammengewogen und unter Rühren innerhalb einiger Minuten gelöst. Zu dieser Mikroemulsionsgrundlage wurden 0.3 mg Astaxanthin zugegeben. Das Carotinoid wurde unter Rühren in der Mikroemulsionsgrundlage innerhalb einiger Stunden gelöst. Es entstand eine klare, orangefarbene Mikroemulsion, die mit jeder beliebigen Menge Wasser mischbar war.

### Beispiel 10

1.6 g Orangenöl (Trägeröl) wurden mit 3.0 g Triglycerinmonolaurat (Emulgator) und 5.4 g demineralisiertem Wasser zusammengewogen und unter Rühren innerhalb einiger Minuten gelöst. Zu dieser Mikroemulsionsgrundlage wurde 1 mg Canthaxanthin gegeben. Das Carotinoid wurde unter Rühren in der Mikroemulsionsgrundlage innerhalb einiger Stunden gelöst. Es entstand eine klare, orangefarbene Mikroemulsion, die mit jeder beliebigen Menge Wasser mischbar war.

### Beispiel 11

1.6 g Orangenöl (Trägeröl) wurden mit 3.0 g Triglycerinmonolaurat (Emulgator) und 5.4 g demineralisiertem Wasser zusammengewogen und unter Rühren innerhalb einiger Minuten gelöst. Zu dieser Mikroemulsionsgrundlage wurden 8 mg Lycopin zugegeben. Das Carotinoid wurde unter Rühren in der Mikroemulsionsgrundlage innerhalb einiger Stunden gelöst. Es entstand eine klare, rote Mikroemulsion, die mit jeder beliebigen Menge Wasser mischbar war.

### Beispiel 12

1.6 g Orangenöl (Trägeröl) wurden mit 3.0 g Triglycerinmonolaurat (Emulgator) und 5.4 g demineralisiertem Wasser zusammengewogen und unter Rühren innerhalb einiger Minuten gelöst. Zu dieser Mikroemulsionsgrundlage wurden 2 mg all-trans-β-Carotin zugegeben. Das Carotinoid wurde unter Rühren in der Mikroemulsionsgrundlage innerhalb einiger Stunden gelöst. Es entstand eine klare, orange-rote Mikroemulsion, die mit jeder beliebigen Menge Wasser mischbar war.

### Beispiel 13

1.6 g Orangenöl (Trägeröl) wurden mit 3.0 g Triglycerinmonolaurat (Emulgator) und 5.4 g demineralisiertem Wasser zusammengewogen und unter Rühren innerhalb einiger Minuten gelöst. Zu dieser Mikroemulsionsgrundlage wurden 30 mg 13-cis-β-Carotin zugegeben. Das Carotinoid wurde unter Rühren in der Mikroemulsionsgrundlage innerhalb einiger Stunden gelöst. Es entstand eine klare, rote Mikroemulsion, die mit jeder beliebigen Menge Wasser mischbar war.

### Beispiel 14

1.6 g Orangenöl (Trägeröl) wurden mit 3.0 g Triglycerinmonolaurat (Emulgator) und 5.4 g demineralisiertem Wasser zusammengewogen und unter Rühren innerhalb einiger Minuten gelöst. Zu dieser Mikroemulsionsgrundlage wurden 90 mg 9-cis-β-Carotin zugegeben. Das Carotinoid wurde unter Rühren in der Mikroemulsionsgrundlage innerhalb einiger Stunden gelöst. Es entstand eine klare, rote Mikroemulsion, die mit jeder beliebigen Menge Wasser mischbar war.

### Beispiel 15

0.6 g Vitamin A-Acetat und 9.4 g Triglycerinmonolaurat (Emulgator) wurden mit 15.0 g demineralisiertem Wasser versetzt und mit einem Magnetrührer unter Rühren innerhalb von ca. 6 Min. auf 60 °C erwärmt. Nach dem Abkühlen entstand eine gelbe Mikroemulsion, die mit beliebigen Mengen Wasser verdünnbar war.

### Beispiel 16

1.0 g Vitamin E-Acetat, 8.0 g Triglycerinmonolaurat (Emulgator) und 1.0 g Imwitor 988 (Caprylsäure-Monodiglycerid der Fa. Hüls, Coemulgator) wurden mit 10.0 g demineralisiertem Wasser mit einem Magnetrührer bei Raumtemperatur gerührt, bis eine Mikroemulsion entstand. Diese Mikroemulsion war mit beliebigen Mengen Wasser verdünnbar.

## Patentansprüche

1. Mikroemulsion vom Typ Oel-in-Wasser, enthaltend mindestens einen Triglycerinmonofettsäureester ausgewählt aus der Gruppe Triglycerinmonolaurat, Triglycerinmonocaprinat und Triglycerinmonocaprylat als Emulgator, und mindestens einen lipophilen Stoff ausgewählt aus der Gruppe Carotinoide, Vitamine A, D, E und K und deren Derivate und mehrfach ungesättigte Fettsäuren als innere Phase.

2. Mikroemulsion nach Anspruch 1, **dadurch gekennzeichnet, dass** der Triglycerinmonofettsäureester Triglycerinmonolaurat oder Triglycerinmonocaprylat ist.

3. Mikroemulsion nach Anspruch 1 oder 2, **dadurch gekennzeichnet, dass** sie zusätzlich einen Coemulgator enthält.

4. Mikroemulsion nach Anspruch 3, **dadurch gekennzeichnet, dass** der Coemulgator Ethanol, Propylenglycol, Transcutol, Polyethlenglycole, Polyglycerine, Monoglyceride oder Lecithin, insbesondere Transcutol, Monoglyceride oder Lecithin, vorzugsweise Lecithin, ist.

5. Mikroemulsion nach einem der Ansprüche 1 bis 4, **dadurch gekennzeichnet, dass** sie zusätzlich mindestens ein Trägeröl enthält.

6. Mikroemulsion nach Anspruch 5, **dadurch gekennzeichnet, dass** das Trägeröl Orangenöl, Palmöl, ein Triglycerid, Squalan, Squalen, Limonen, Isopropylmyristat oder Isopropylpalmitat, insbesondere Orangenöl, Squalan, Limonen, vorzugsweise Orangenöl, ist.

7. Mikroemulsion nach einem der Ansprüche 1 bis 6, **dadurch gekennzeichnet, dass** sie als Vitamin Tocopherol und/oder Vitamin A, insbesondere Vitamin A Palmitat, enthält.

8. Mikroemulsion nach einem der Ansprüche 1 - 7, **dadurch gekennzeichnet, dass** der Anteil an lipophilem Stoff, insbesondere an Tocopherol, 1-20 Gew.%, an Emulgator 10-99 Gew.% und gegebenenfalls an Coemulgator 1-89 Gew.% beträgt, wobei vorzugsweise das Verhältnis von Emulgator zu Coemulgator etwa 50:50 Gew.% ist.

9. Mikroemulsion nach einem der Ansprüche 1 bis 8, **dadurch gekennzeichnet, dass** sie als Carotinoid mindestens eines aus der Gruppe all-trans-β-Carotin, 9-cis-β- Carotin, 13-cis-β-Carotin, Apocarotinal, Astaxantin, Canthaxanthin, Crocetin und Lycopin, vorzugsweise mindestens ein β-Carotin, enthält.

## Claims

1. A microemulsion of the oil-in-water type, containing at least one triglycerol monofatty acid ester selected from the group of triglycerol monolaurate, triglycerol monocaproate and triglycerol monocaprylate as the emulsifier and at least one lipophilic substance selected from the group of carotenoids, vitamins A, D, E and K and their derivatives and polyunsaturated fatty acids as the internal phase.

2. A microemulsion according to claim 1, **characterized in that** the triglycerol monofatty acid ester is triglycerol monolaurate or triglycerol monocaprylate.

3. A microemulsion according to claim 1 or 2, **characterized in that** it additionally contains a co-emulsifier.

4. A microemulsion according to claim 3, **characterized in that** the co-emulsifier is ethanol, propylene glycol, transcutol, polyethylene glycols, polyglycerols, monoglycerides or lecithin, especially transcutol, monoglycerides or lecithin, preferably lecithin.

5. A microemulsion according to any one of claims 1 to 4, **characterized in that** it additionally contains at least one carrier oil.

6. A microemulsion according to claim 5, **characterized in that** the carrier oil is orange oil, palm oil, a triglyceride, squalane, squalene, limonene, isopropyl myristate or isopropyl palmitate, especially orange oil, squalane [or] limonene, preferably orange oil.

7. A microemulsion according to any one of claims 1 to 6, **characterized in that** it contains tocopherol and/or vitamin A, especially vitamin A palmitate, as the vitamin.

8. A microemulsion according to any one of claims 1-7, **characterized in that** the content of lipophilic substance, especially of tocopherol, is 1-20 wt.%, the content of emulsifier is 10-99 wt.% and the content of co-emulsifier (optional) is 1-89 wt.%, with the ratio of emulsifier to co-emulsifier preferably being about 50:50 wt.%.

9. A microemulsion according to any one of daims 1 to 8, **characterized in that** it contains at least one from the group of all-trans-β-carotene, 9-cis-β-carotene, 13-cis-β-carotene, apocarotenal, astaxanthin, canthaxanthin, crocetin and lycopene, preferably at least one β-carotene, as the carotenoid.

## Revendications

1. Microémulsion du type huile dans l'eau, contenant au moins un monoester d'acide gras de triglycérine, choisi parmi le groupe du monolaurate de triglycérine, du monocaprinate de triglycérine et du monocaprylate de triglycérine à titre d'émulsifiant et au moins une substance lipophile choisie parmi le groupe des caroténoïdes, des vitamines A, D, E et K et de leurs dérivés et des acides gras plusieurs fois insaturés à titre de phase interne.

2. Microémulsion selon la revendication 1, **caractérisée en ce que** le monoester d'acide gras de triglycérine est le monolaurate de triglycérine ou le monocaprylate de triglycérine.

3. Microémulsion selon la revendication 1 ou 2, **caractérisée en ce qu'**elle contient en plus un co-émulsifiant.

4. Microémulsion selon la revendication 3, **caractérisée en ce que** le co-émulsifiant est l'éthanol, le propylène glycol, le transcutol, les polyéthylène glycols, les polyglycérines, les monoglycérides ou la lécithine, en particulier le transcutol, les monoglycérides ou la lécithine, de préférence la lécithine.

5. Microémulsion selon l'une des revendications 1 à 4, **caractérisée en ce qu'**elle comprend en plus une huile support.

6. Microémulsion selon la revendication 5, **caractérisée en ce que** l'huile support est l'huile d'oranger, l'huile de palme, un triglycéride, le squalane, le squalène, le limonène, le myristate d'isopropyle ou le palmitate d'isopropyle, en particulier l'huile d'oranger, le squalane, le limonène, de préférence l'huile d'oranger.

7. Microémulsion selon l'une quelconque des revendications 1 à 6, **caractérisée en ce qu'**elle comprend à titre de vitamine du tocophérol et/ou de la vitamine A, en particulier le palmitate de vitamine A.

8. Microémulsion selon l'une quelconque des revendications 1 à 7, **caractérisée en ce que** la part de substance lipophile, en particulier de tocophérol, va de 1 à 20 % en poids, la part d'émulsifiant va de 10 à 99 % en poids et éventuellement la part de coémulsifiant de 1 à 89 % en poids, le rapport de l'émulsifiant sur le coémulsifiant étant d'environ 50 / 50 % en poids.

9. Microémulsion selon l'une quelconque des revendications 1 à 8, **caractérisée en ce qu'**elle comprend à titre de caroténoïde au moins un élément parmi le groupe all-trans-β-carotène, 9-cis-β-carotène, 13-cis-β-carotène, apocarotinal, astaxantine, canthaxantine, crocétine et lycopine, de préférence au moins un β-carotène.
